Europäisches Patentamt

European Patent Office

Office européen des brevets

(11) Publication number: **0 067 697**
**B1**

## (12) EUROPEAN PATENT SPECIFICATION

(45) Date of publication of patent specification: **18.09.85**

(51) Int. Cl.⁴: **C 12 N 5/00**

(21) Application number: **82303067.1**

(22) Date of filing: **14.06.82**

(54) **Cell culture method.**

(30) Priority: **15.06.81 JP 91733/81**

(43) Date of publication of application:
**22.12.82 Bulletin 82/51**

(45) Publication of the grant of the patent:
**18.09.85 Bulletin 85/38**

(84) Designated Contracting States:
**AT BE CH DE FR GB LI NL**

(56) References cited:
**EP-A-0 049 632**

**BIOLOGICAL ABSTRACTS, vol.70, 1980, abstract no.49413 D.M. FALALEEVA et al.: "Biosynthesis of alpha-fetoprotein in tissue culture of human embryo"**

**CHEMICAL ABSTRACTS, vol.94, 1981, page 302, abstract no.27628p, Columbus, Ohio (US) P.M- BRADLEY: "Co-culture of carrot cells and a green alga on medium deficient in nitrogen"**

(73) Proprietor: **CHLORELLA INDUSTRY CO., LTD**
**5th Floor, Toyokuni Building No. 4-6 Shiba Daimon 2-chome**
**Minato-ku Tokyo (JP)**

(72) Inventor: **Kumamoto, Shoichiro**
**No. 412-2, Oaza Okuda**
**Oita-shi Oita (JP)**

(74) Representative: **Perry, Robert Edward et al**
**GILL JENNINGS & EVERY 53-64 Chancery Lane**
**London WC2A 1HN (GB)**

Courier Press, Leamington Spa, England.

# Description

The present invention relates to a method of cell culture, and in particular of human cells.

Human cell tissues have been sub-cultured for a wide variety of biological studies, including medicine and pharmacology, as an effective and practicable means of research. For this reason, the technique has been applied in research, e.g. in elucidating the mechanism of cancer and in cytofusion.

As described in EP—A—0049632, it has previously been considered almost always necessary, for the sub-culture of animal tissue, to use a serum of, for example, an adult, neonate or foetus of a subject which may be bovine or a horse, chicken or rabbit, in addition to a chemically-defined synthetic culture medium comprising, for example, amino-acids, vitamins and/or minerals. The need for a serum is based on the fact that it contains a cell multiplicative growth factor (a substance other than the general nutrient materials); therefore, multiplication of animal tissue cells is impossible without serum. Sera are, however, unstable and expansive.

It has been found that, when sub-culturing normal human cells *in vitro*, even when using a basal culture medium containing animal serum (ordinarily a cattle foetus serum), the maximum number of cycles is about 50—60. If a larger number of cycles is used, altered morphologies and changed hereditary genes are observed; alternatively, no further cell segmentation was found to be possible, the culture tending to biolysis.

EP—A—0049632 discloses and claims a cell culture method which comprises cultivating cells in a culture medium comprising microalgae.

According to the present invention, a cell culture method comprises cultivating human cells in a culture medium comprising microalgae in the form of an aqueous extract.

As used in this specification, the term "microalgae" means unicellular algae or their near relatives such as, for example, Chlorella, Scenedesmus or Spirulina. The algae may be naturally-occurring of cultivated.

For use in the culture medium, the cells of one or more types of microalgae are extracted, e.g. with water itself or an aqueous solution of an acid, base or organic solvent. For extraction, algae cells are brought into contact with an aqueous solution which may be at ambient or elevated temperature. Hot water extraction is preferred. By way of example, algae cells are suspended in water in an amount of 1 to 1000 grams algae (dry weight) per liter of water and are maintained at 50 to 150°C for 0.5 to 120 minutes, preferably at about 100°C for more than a minute, and are then separated from water by, for example, centrifugation. The resultant extract may be purified, if desired, by any suitable technique such as gel filtration or dialysis.

The extract usually contains sugars, proteins, polysaccharides and nucleic acids at least, the given components having molecular weights in the range of from 1,000 to 1,000,000. Such extracts can exhibit multiplication-promoting activity in cultivating human cells. The term "cells" will be understood herein to include tissue.

The extract can be used per se, as a fraction obtained by molecular weight fractionation thereof, or in the form of a concentrate or dry powder obtained by, for example, freeze-drying or spray-drying. The preferred forms for use in the invention are powders and a high molecular fraction containing sugars, proteins and polysaccharides.

The culture process of the invention may follow the procedures of, and use the same basic culture media as are used in, conventional tissue culture. Thus, a known synthetic culture medium may be used. The medium may contain, for example, amino-acids and/or vitamins as the basal culture medium, to which a microalgae extract as described above is added, and whereon the cultivation of human cells is performed, under aseptic conditions. The culture medium may also contain serum, e.g. animal serum, but the amount which is needed may be no more than 10% of that previously considered to be necessary in cultivating animal cells; for example, a culture medium used in the present invention may contain no more than 1% of serum.

The amount of the microalgae present in the medium may be from 0.1 to 400, and is preferably from 1 to 100 mg (dry weight) of extracted matter per litre of the medium. The use of such amounts of microalgae, in the cultivation of human cells, apparently makes it possible to accelerate cell multiplication and also to maintain normal successive cultivation without any morphological or genetic mutation. These effects can be achieved using considerably lower amounts of animal serum than have previously been considered necessary. The reasons for the advantageous effects observed in operation of the present invention are unclear, but it is theorised that cell metabolism is stimulated by the action of some substance, probably of high molecular weight, such as glycoprotein or polysaccharide, in the microalgae.

The following Example illustrates the invention.

## Example 1

A fraction (Fraction $A_1$) was obtained in the manner as described in Example 1 of European Patent Specification No. 49632. $1 \times 10^2$ of human fibroblasts were cultivated in a culture medium prepared by adding 10% of cattle foetus serum to a sector of a basic culture medium (Sector C) as defined in European Patent Specification No. 49632.

In a similar manner, $1 \times 10^2$ of this cell strain were cultivated in a culture medium prepared by adding 1% of cattle foetus serum and 10 mg/l Fraction $A_1$ to the culture medium; the culture sector is denoted as Sector T. It was observed that, after 50—60 generation cycles (in about 10 months), normal cell segmentation no longer occurred, and that there was cell biolysis, in Sector C. In Sector T, sub-culturing was maintained successfully for 90—120 generations, without morphological or genetic mutation.

**Claims**

1. A cell culture method, which comprises cultivating human cells in a culture medium, characterised in that the medium comprises microalgae in the form of an aqueous extract.

2. A method according to claim 1, in which the microalgae are selected from Chlorella, Scenedesmus and Spirulina.

3. A method according to claim 1 or claim 2, in which each litre of the medium comprises from 0.3 to 400 mg of the microalgae.

4. A method according to any preceding claim, in which the medium additionally comprises animal serum.

5. A method according to any preceding claim, in which the cells are normal tissue or cancer cells.

**Patentansprüche**

1. Zellkulturverfahren, welches ein Vermehren von Humanzellen, in einem Kulturmedium umfaßt, dadurch gekennzeichnet, daß das Medium Mikroalgen in Form eines wässerigen Extraktes umfaßt.

2. Verfahren nach Anspruch 1, worin die Mikroalgen unter Chlorella, Scenedesmus und Spirolina ausgewählt sind.

3. Verfahren nach Anspruch 1 oder 2, worin jeder Liter des Mediums von 0,3 bis 400 mg der Mikroalgen umfaßt.

4. Verfahren nach einem der vorstehenden Ansprüche, worin das Medium zusätzlich tierisches Serum umfaßt.

5. Verfahren nach einem der vorstehenden Ansprüche, worin die Zellen Normalgewebezellen oder Krebszellen sind.

**Revendications**

1. Un procédé de culture de cellules, qui consiste à cultiver des cellules humaines dans un milieu de culture, caractérisé en ce que le mileu comprend des microalgues sous la forme d'un extrait aqueux.

2. Un procédé selon la revendication 1, dans lequel les microalgues sont choisies parmi Chlorella, Scenedesmus et spirulina.

3. Un procédé selon la revendication 1 ou ou 2, dans lequel chaque litre du milieu comprend de 0,3 à 400 mg de microalgues.

4. Un procédé selon l'une quelconque des revendications précédentes, dans lequel le milieu comprend en outre un sérum animal.

5. Un procédé selon l'une quelconque des revendications précédentes, dans lequel les cellules sont des cellules de tissu normal ou des cellules canéreuses.